# EUROPEAN PATENT APPLICATION

(11) **EP 1 416 053 A1**
(43) Date of publication of application: **06.05.2004**
(21) Application number: 02079423.6
(22) Date of filing: 22.10.2002
(51) Int. Cl.: C12P 7/56

(54) **Separation of biomass from lactic acid containing fermentation products by means of flocculation**

(71) Applicant: Purac Biochem B.V., 4200 AA Gorinchem (NL)
(72) Inventor: Baets, Peter Johannes Marie, 4201 HB Gorinchem (NL); Coldenhoff, Ernst Robert, 4241 DS Arkel (NL)
(74) Representative: van Westenbrugge, Andries

(57) **Abstract**

The present invention is in the field of the preparation of lactic acid by means of fermentation, more particularly it is directed to processes to separate the biomass from the lactic acid-containing fermentation product by means of flocculation.

We have found that for the separation of biomass from lactate and lactic acid-containing fermentation broth the following process is very convenient and suitable:
a) Subjecting the fermentation broth to an alkalifying step,
b) adding one or more flocculants, and
c) separating the biomass flocs from the lactic acid-containing fermentation broth.

With this process even bacteria-based fermentation broths can be suitably handled.

## Description

The present invention is in the field of the preparation of lactic acid by means of fermentation, more particularly it is directed to processes to separate the biomass from the lactate and lactic acid-containing fermentation product by means of flocculation.

Lactic acid, its salts and esters have long been used as food additive and in various chemical and pharmaceutical applications. More recently, lactic acid has been used in the making of biodegradable polymers both as a replacement for present plastic materials as well as various new uses were biodegradability is needed or desired such as for medical implants and slow release drugs. Accordingly there is an ever increasing demand for improved and economically viable lactic acid processes. This invention provides an improvement of the conventional lactic acid production processes via fermentation.

The production of lactic acid is commonly carried out by fermentation by means of a micro-organism such as bacteria, yeasts and fungi. The fermentation substrate consists of carbohydrates together with suitable mineral and proteinaceous nutrients. After fermentation, the lactate and lactic acid-containing fermentation product must be separated from the biomass. Said lactic -acid-containing- fermentation product is in the liquid form (i.e. liquid or in solution). Usually this biomass is separated by means of filtration, centrifuging, flocculation, coagulation, flotation or combinations thereof. This is for instance described in WO 01/38283 wherein a continuous process for the preparation of lactic acid by means of fermentation is described. Especially when the fermentation is carried out by means of bacteria, said biomass separation is not easy. Because of the small solid particles in the biomass filtration *per se* is not possible.

We have found that for the separation of biomass from lactate and lactic acid-containing fermentation broth the following process is very convenient and suitable:
a) Subjecting the fermentation broth to an alkalifying step,
b) adding one or more flocculants, and
c) separating the biomass flocs from the lactic acid-containing fermentation broth.

With this process even bacteria-based fermentation broths can be suitably handled.

In step a) the pH of the fermentation broth is increased to above 10. Said alkalifying step is performed maintaining the temperature of the fermentation broth between 50 and 100 °C. This pH increase may be obtained by adding alkaline compounds, either as solids, in solution or in suspension. Examples of suitable alkaline compounds are calcium (hydr)oxide, sodium hydroxide , potassium hydroxide, lithium hydroxide, magnesium (hydr)oxide, ammonium compounds such as ammonium hydroxide, quaternary ammonium hydroxides and amines such as mono ethanol amine, triethanol amine, etcetera to the fermentation broth. The use of calcium (hydr)oxide is preferred because it ensures a lactic acid-containing product of high clarity.

Optionally, the mixture obtained in the alkalifying step is aged at a temperature between 50-100 °C up to 25 hours. When an ageing step is used the ageing time preferably is above 8 hours, because this ageing time ensures proper flocculation and low metal and silica concentration in the lactic-acid containing fermentation product.

It was found, however, that omitting the ageing step altogether and controlling the alkalifying residence time to very short periods gave extremely good results. The alkalifying residence time is defined as the time between increasing the pH to above 10 and separating the biomass flocs. When setting the alkalifying residence time to between 1 second and 4 hours it was found that proper flocculation was ensured, the amount of polysaccharide in the final lactate and lactic acid-containing fermentation product was very low, and the final lactate and lactic acid-containing fermentation product had a very high clarity. Even better results were obtained when setting the alkalifying residence time to between 1 and 60 seconds. This has advantages because the subsequent purification steps of the lactic acid may be less stringent or may even be omitted. Conventional subsequent purification steps are distillation including short path distillation and vacuum distillation, crystallisation, electrodialysis, extraction, carbon treatment, ion exchange and combinations thereof.

In order to ensure a very short alkalifying residence time, the alkalifying is preferably conducted in-line. With in-line alkalifying residence times of between 1 and 30 seconds, even between 1 and 15 seconds, may be reached.

Suitable flocculants are known in the art and need no further elucidation here. In the process according to the invention the use of orthophosphoric acid and polymer flocculant such as anionic polyacrylic amides and copolymers thereof or ammonium acrylates are preferred. Other suitable polymeric flocculants are described in Kirk-Othmer, Encyclopedia of Chemical Technology 3^{rd}. Ed. Volume 10, pages 502-503. Usually about 50 to 500 ppm orthophosphoric acid is used, based on the total weight of the fermentation broth, preferably about 100-300 ppm is used. The amount of polymer may range from 1-2000 ppm, preferably between 10 -500 ppm, more preferably between 30-70 ppm. Also combinations of flocculants may be used, with a combination of orthophosphoric acid and polymeric flocculant being preferred. During the addition of the flocculants a fermentation broth temperature of between 50 and 80 °C, preferably between 60-70 °C, is maintained. In order to control the alkalifying residence time properly, it is preferred to add the flocculants in-line. The order of addition of the various flocculants is not critical and any order of addition may be used. The flocculants are usually added in solution, the concentration of the solution being adapted to obtain the desired viscosity.

Both steps a) and b) are preferably conducted with agitation. The agitation, however, should not be as severe as to break up the biomass flocs Said agitation may be accomplished with static mixers, pumps, etcetera. Steps a) and b) may also be combined.

The separation of the biomass flocs from the lactate and lactic-acid-containing fermentation broth may be done with any liquid-solid separation method such as filtration, for instance with a drum filter or belt filter, sedimentation, for example with a static sedimentation tank, sedimentation centrifuge or a cyclone. These separation methods are known in the art and need no further elucidation here.

Optionally, the biomass precipitate is washed once or more after separation and subjected to one or more additional flocculant addition steps, followed by separation of the biomass precipitate to increase the overall process lactic acid yield.

The invention is further illustrated by means of the following examples, which are not to be construed as being limitative.

### Example 1

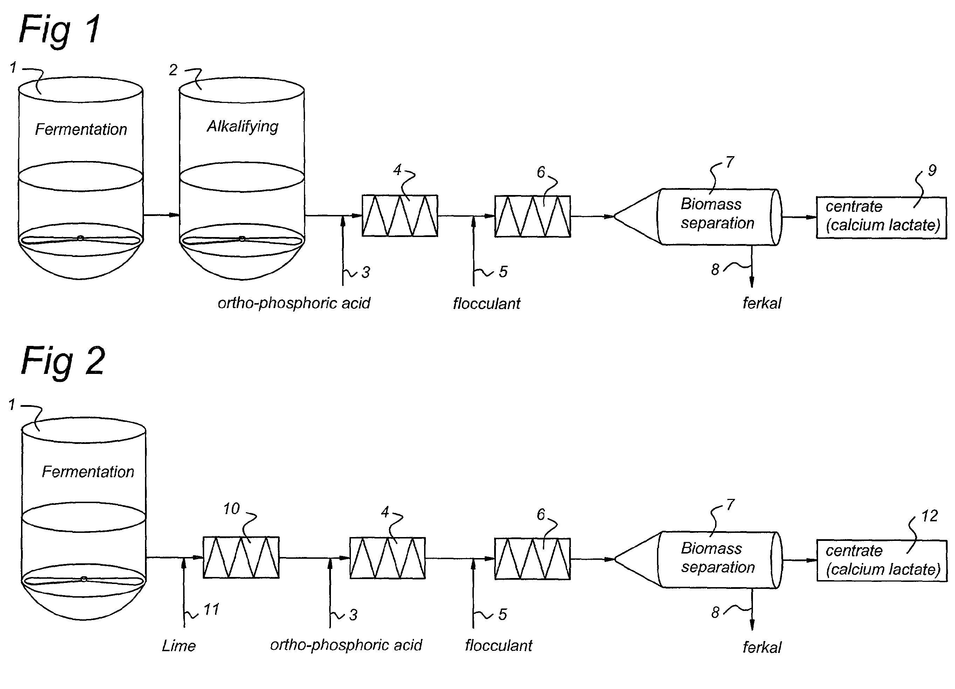

A process set-up for biomass removal consisting of an alkalifying vessel (2) and an inline ortho-phosphoric acid (3) and flocculant (5) dose according to Figure 1 was used. In this set-up sugar free fermentation broth (1) obtained from bacteria fermentation using lactic acid-producing bacteria with a temperature of 70°C was pumped into the alkalifying vessel (2). In this vessel a 25% calcium hydroxide suspension was added to the broth. Being alkaline, for minimal 6-7 till max. 14 hrs, the broth was transferred (passing mixers (4) and (6) into the direction of the decanter (7) and meanwhile mixed with 160 ppm ortho-phosphoric acid (OPA) and 60 ppm polymeric flocculant, anionic polyacrylic amide, inline. In the decanter the broth is easily split into biomass (8) and lactic acid-containing fermentation product (9), hereinafter referred to as product 1.

### Example 2

In this example a set-up was used according to Figure 2. Herein the alkalifying vessel is skipped and an extra static mixer (10) is implemented. A 25 % calcium hydroxide suspension (11) was dosed inline. After dosing, the fermentation broth obtained from bacteria fermentation using lactic acid-producing bacteria was mixed with the static mixer (10). Just after this static mixer 160 ppm orthophosphoric acid was added and the flow was mixed again by means of mixer (4). To the mixed broth a solution of polymeric flocculant, 60 ppm anionic polyacrylic amide, was added and also this was mixed inline using static mixer (6). All mixers are Sulzer SMI-W DN25 static mixers. The alkalifying residence time was about 15 seconds. Passing this mixer the flow entered the decanter (7). In the decanter it is split into biomass (8) and lactate and lactic acid-containing fermentation product (12), hereinafter referred to as product 2.

When comparing the polysaccharide content in product 1 with the polysaccharide content of product 2, the product of example 2 has a polysaccharide content of about 50 % of the polysaccharide content of the product of example 1. The metal content of product 1 was lower than of product 2. Both products were clear, with product 2 having a very high clarity.

## Claims

1. Process for the separation of biomass from lactate and lactic acid-containing fermentation product present in a fermentation broth by:
a) subjecting the fermentation broth to an alkalifying step,
b) adding one or more flocculants, and
c) separating the biomass flocs from the fermentation broth.

2. Process according to claim 1 wherein in the alkalifying step the pH of the fermentation broth is increased to above 10.

3. Process according to claim 1 or 2 wherein the mixture obtained in the alkalifying step is aged at a temperature between 50-100 °C for a period of time up to 100 hours.

4. Process according to claim 3 wherein the ageing time is above 8 hours.

5. Process according to claim 1, wherein the alkalifying residence time is between 1 second and 4 hours.

6. Process according to claim 1, wherein the alkalifying residence time is between 1 and 60 seconds.

7. Process according to claim 1 wherein the alkalifying residence time is between 1 and 30 seconds, preferably between 1-15 seconds.

8. Process according to any one of the preceding claims wherein the flocculant is orthophosphoric acid.

9. Process according to any one of the preceding claims wherein the flocculant is a polymeric flocculant.

10. Process according to any one of the preceding claims wherein steps a) and b) are conducted with agitation.

11. Process according to any one of the preceding claims wherein steps a) and b) are combined.

12. Process according to any one of the preceding claims wherein the alkalifying step is conducted in-line.

13. Process according to any one of the preceding claims wherein step b) is conducted in-line.

14. Process according to any one of the preceding claims wherein the biomass precipitate is subjected to one or more washing steps and one or more additional flocculant addition steps, followed by separation of the biomass precipitate.
